# EUROPEAN PATENT SPECIFICATION

(11) **EP 4 252 619 B1**
(45) Date of publication and mention of the grant of the patent: **18.11.2026**
(21) Application number: 23158022.6
(22) Date of filing: 22.02.2023
(51) Int. Cl.: A61B 1/018, A61B 1/01, A61B 1/00, A61B 1/307

(54) **HAND-HELD SURGICAL INSTRUMENT AND SHAFT FOR HAND-HELD SURGICAL INSTRUMENT**
CHIRURGISCHES HANDINSTRUMENT UND SCHAFT FÜR CHIRURGISCHES HANDINSTRUMENT
INSTRUMENT CHIRURGICAL PORTATIF ET TIGE POUR INSTRUMENT CHIRURGICAL PORTATIF

(30) Priority: 30.03.2022 US 202263325400 P
(43) Date of publication of application: 04.10.2023
(73) Proprietor: Olympus Winter & Ibe GmbH, 22045 Hamburg (DE)
(72) Inventor: Schmuck, Irina, 25746 Heide (DE); Schulz, Kevin Alexander, 22889 Wilstedt (DE); Schröder, Bastian, 22589 Hamburg (DE); RÜHS, Andreas, 22926 Ahrensburg (DE)
(74) Representative: Hoener, Matthias

(56) References cited:
- DE-A1- 102016 003 175
- DE-B4- 102004 043 460

## Description

The invention relates to a shaft for a hand-held surgical instrument according to claim 1.

Hand-held surgical instruments, such as endoscopes, resectoscopes, cystoscopes and the like, consist essentially of a device or main body and a tube-like shaft. The shaft allows various instruments to be inserted into a person's body for medical treatment of the person. The instruments may be an optic or, in the case of a resectoscope, an electrode array, or through articles such as a catheter, wires or the like. In this regard, it is intended for most instruments to be movable within the shaft parallel to the longitudinal direction of the shaft. The space in the shaft that is not occupied by the optics is referred to as the working channel. The other instruments can be inserted into the shaft or body through this working channel.

For example, in order to carry out the treatment of body tissue by means of a catheter, the treatment area is covered by an optical system during the deployment of the catheter. For this purpose, both the rod-shaped optics and the catheter are guided through the shaft and thus through the tip of the shaft. To ensure that the optic is always aligned in a predefined position relative to the shaft and thus obtains an ideal view of the treatment site, it is fixed within a hole or a bore in the tip of the shaft. To do this, the optic is pushed into the shaft from the proximal end before treatment begins and positioned with its distal end in the distal bore of the shaft. The area below the optic is used to insert the other through articles, such as the catheter, into the shaft. This working channel is also formed by the outer wall of the optic.

This aforementioned positioning of the optics and the through article within the shaft works particularly well when the cross-section of the working channel is small compared to the diameter of the optics. In this case, the cross-section of the shaft can be drop-shaped. When the optic is inserted into this drop-shaped shaft, the optic automatically positions itself in the wider area of the shaft and is thereby guided into the bore with the distal end. Subsequently, the through article is guided into the free working channel below the optic. The surgeon thus knows how the optics and also the distal end of the through article are positioned relative to the shaft tip. In addition to the hole, the shaft tip has a second opening through which the distal end of the through article is guided. This second opening may be slightly downward, i.e., away from the bore, if necessary.

It proves to be problematic if the diameter of the working channel is larger in comparison to the optics than in the case described above. If the working channel has a similar diameter to the optics, the cross-section of the shaft must be designed accordingly. The cross-section of the shaft can be oval or circular in shape. However, with such a cross-sectional shape, the optic is no longer automatically guided into the bore when it is pushed into the shaft, but sags and thus blocks the working channel. In addition, the distal end of the optic can become misaligned in the distal opening of the working channel, which leads to a malfunction or time delay in the use of the surgical handpiece.

A prior art document is document DE 10 2016 003 175 A1. It discloses a shaft for a hand-held surgical endoscope comprising an inner contour with distinct areas for an optic and a working channel. To secure the optic against lateral displacement, the inner wall of the shaft features inwardly formed longitudinal beads. However, these guiding beads run strictly parallel to the longitudinal axis of the shaft over essentially its entire length to maintain a constant internal cross-section.

The present invention is directed to providing a shaft and a hand-held surgical device in which an optic and a through article can be positioned in the shaft in a simple and reliable manner.

A solution to this task is described by the features of claim 1. Accordingly, it is provided that at least one bar is arranged on an inner wall of the shaft through which the optic is guided from the proximal end of the shaft into the bore of the tip of the shaft. The at least one bar is formed such that the distal tip of the optic is guided into the bore when the optic is pushed into the shaft, regardless of the shape of the cross-section of the shaft. This makes it possible to ensure that the optic is always guided into the correct position, namely into the bore of the shaft tip. Once the optics are positioned inside the shaft, the other through articles can also be guided in a simple and reliable manner through the working channel then formed into the shaft tip.

Preferably, the invention provides for two opposing bars to be positioned on the inner wall of the shaft. By using two bars positioned on opposite sides of the inner wall within the shaft, the positioning of the optic within the shaft can be made even more reliable. This symmetrical arrangement of the bars allows the optics to be guided into the bore of the shaft tip with a high degree of precision. This forced guidance can eliminate the possibility of the optic being inserted into the shaft in a way that was not intended.

A particularly preferred embodiment of the invention may provide for the bar to extend in a U-shape along the inner wall of the shaft, with the open legs of the bar facing towards the bore. This angled U within the shaft provides a ramp for the distal end of the optic to be guided into the bore. This configuration of the bar allows the optic to be guided into the correct position within the tip of the shaft, particularly in the case of oval or circular cross-sections of the shaft. It is essential in this regard that the at least one bar is oriented obliquely with respect to a longitudinal axis of the shaft, namely in the direction of the bore. This oblique orientation of the at least one bar relative to the bore and the longitudinal axis of the shaft causes the optics to slide on this ramp-like arrangement directly into the bore of the shaft tip.

In particular, it may also be provided that a cross-section of the at least one bar is concave. This concave shape of the bar allows the distal end of the optic to be received without the optic being able to snag within the shaft. Also, the through article to be subsequently inserted cannot jam or snag on the concave shape of the at least one bar. The concave shape continuously increases the wall thickness of the shaft at the locations of the at least one bar. Due to this continuous expansion of the wall thickness, the at least one bar does not form a mechanical barrier for any objects or articles to be inserted into the shaft. The through articles that encounter the bar during insertion into the shaft are directed back to the center of the shaft by the concavity. Blocking of the through article within the shaft is thus prevented.

The invention may further provide that the distance between the two bars or between the two legs of the U-shaped bar is 1 mm to 4 mm, in particular 2 mm to 4 mm, preferably 3.6 mm, or less than a diameter of the optic. Furthermore, it may be provided that the distance between the two bars or between the two legs of the U-shaped bar is greater than a diameter of the through article. This configuration of the bars ensures that the optic is guided upward into the bore and the through article remains in the lower section or working channel. Because the distance between the bars is greater than the dimension of the through article, it cannot get caught inside the working channel or on the two bars. The surgeon can therefore insert the optic first and then the through article into the shaft in a very simple and reliable manner and without having to take special care with regard to possible blocking.

Further, according to the invention, it may be provided that a dividing wall between the bore and the working channel is such that the through article is guided downwards, i.e. away from the bore. This dividing wall can also have a concavity in order to guide the article into the correct position within the shaft tip in a particularly reliable manner, and also to make it easy and reliable to pull the through article out of the shaft.

Another advantageous embodiment of the invention may provide that the at least one bar is an end face of a widening of the wall of the shaft tip. By the fact that the wall of the shank, viewed in the distal direction, is also widened after the at least one bar, or that the wall has the thickness of the bar, the through article cannot block or get caught on the bar when it is pulled out of the shank. This makes the wall thickness of the working channel greater than the wall thickness of the bore and/or the shaft.

A hand-held surgical instrument for solving the aforementioned task is described by the features of claim 12. Accordingly, it is provided that a hand-held surgical instrument, in particular a cystoscope, a resectoscope or the like, has a shaft according to at least one of claims 1 to 11.

A preferred embodiment of the invention is described in more detail below with reference to the drawing. In this show:
- Fig. 1: a schematic representation of a hand-held surgical instrument,
- Fig. 2: a sectional view through the shaft tip according to Fig. 1,
- Fig. 3a: a perspective view of a shaft tip,
- Fig. 3b: another perspective view of the shaft tip,
- Fig. 4: a detail enlargement of the illustration according to Fig. 2b,
- Fig. 5: a sectional view through the shaft tip,
- Fig. 6a: a schematic representation of a position finding of an optical system,
- Fig. 6b: a further schematic representation of the positioning of the optics,
- Fig. 6c: a further schematic representation of the positioning of the optics,
- Fig. 7a: schematic representation of a position finding of a through article,
- Fig. 7b: another schematic representation of the position finding of the through article, and
- Fig. 7c: an additional schematic representation of the position finding of the through article.

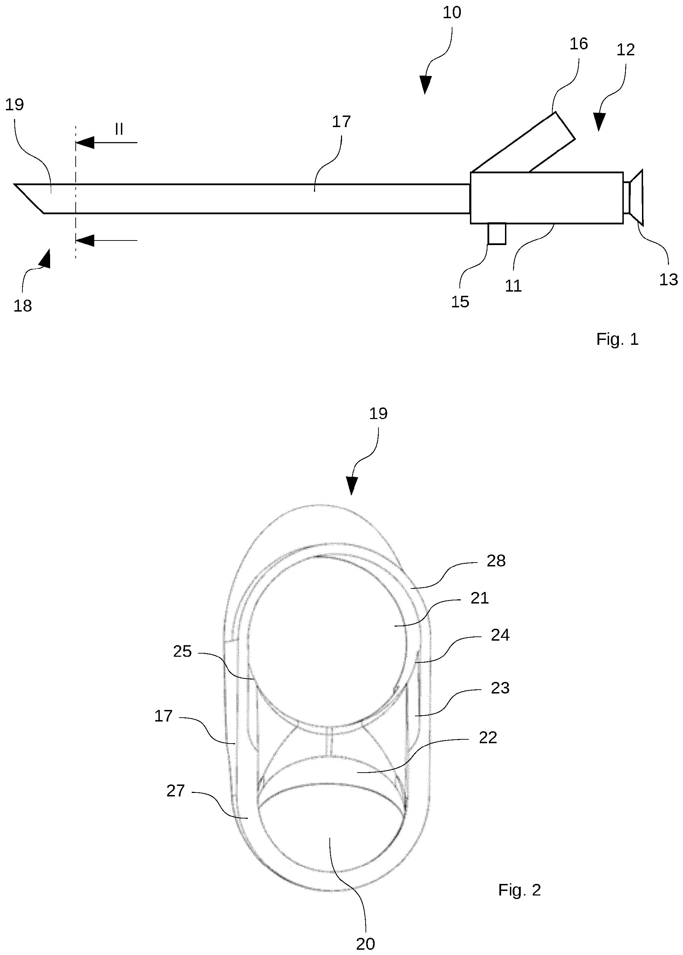

In Fig. 1, a hand-held surgical instrument 10 is shown in a highly schematized form for better illustration. This hand-held surgical instrument 10 has a device body 11, which can also be designed as a handle unit. Furthermore, an ocular 13 for an optical system 14 is arranged at a proximal end 12 of the device body 11. In addition, the device body 11 shown here purely schematically has two connections or ports 15, 16. These connections 15, 16 can serve both to supply the hand-held surgical instrument 10 with electrical energy, light, a fluid or as a port for further instruments.

A tube-like shaft 17 is arranged at an end opposite the proximal end 12 of the device body 11. This shaft 17 is elongated in shape and serves to be inserted into the patient with a distal end 18 of the shaft 17 or with a tip 19 of the shaft. This shaft 17 is also shown in Fig. 1 in a highly schematized form and serves only for a better understanding of the invention described herein. It should be expressly noted that such a shaft 17 can also be coupled with other surgical devices. Thus, the oblique design of the shaft tip 19 of the shaft 17 is also to be regarded as strongly schematized. Indeed, it is quite possible for the shape of the bevel to differ greatly from that shown in Fig. 1.

In the hand-held surgical instrument 10 described herein, it is contemplated that both an optic, which is not shown, and a through article, which is not shown, which may be a catheter, for example, will pass through the shaft 17. In doing so, the two aforementioned articles are pushed into the shaft 17 from the proximal end 12 of the hand-held instrument 10 until the rod-shaped optic, which may be a light guide or a rod lens system, protrudes with its distal end from the distal end 18 of the shaft 17. Similarly, the through article is moved through the shaft 17 until it protrudes from the distal end 18 of the shaft 17. In doing so, the optic and the through article are mounted parallel to each other as well as parallel to a longitudinal axis of the shaft 17. In preparation for the treatment or operation to be performed, the optic is first inserted into the shaft 17. In doing so, the optic is positioned in an upper region of the shaft 17. The space within the shaft 17 that forms below the optic is referred to as the working channel 20 and is used to accommodate the through article. In the embodiment described herein, the dimension of the cross-section of the working channel 20 is approximately the diameter of the optic. Accordingly, the cross-section of the shaft 17 is oval. However, it is equally conceivable that the cross-section of the shaft 17 has a different shape.

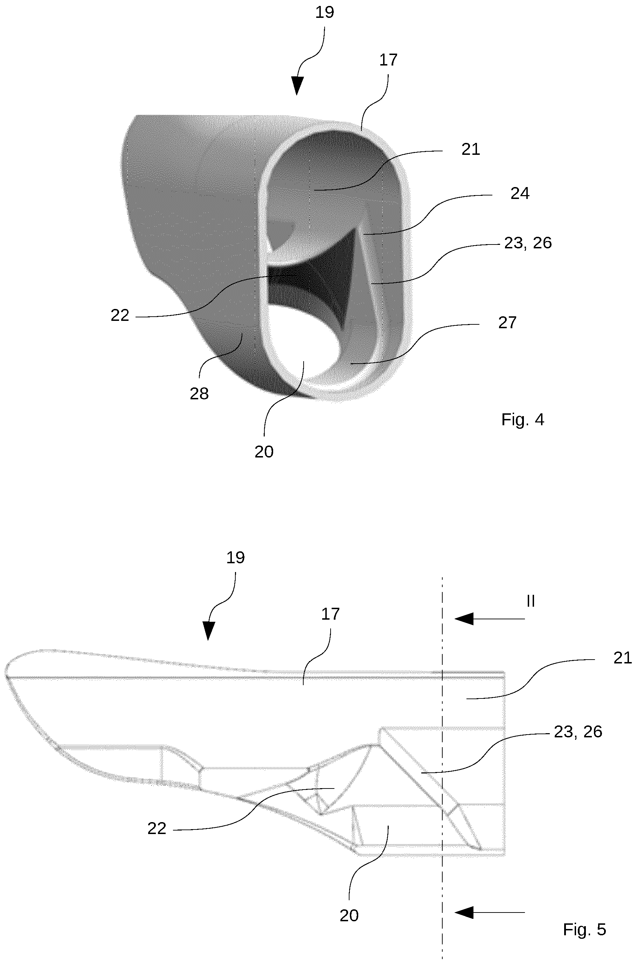

In Fig. 2, a sectional view through the distal end 18 of the shaft 17 or through the shaft tip 19 is shown, looking in the distal direction. Through this sectional view, the oval cross-sectional shape of the shaft 17 becomes particularly clear. While a bore 21 for receiving the distal end of the optic is located in the upper region of the shaft 17, the working channel 20 for receiving the through article is arranged below it. Between the bore 21 and the working channel 20 is a dividing wall 22, which separates the two openings from each other (Fig. 5). While the bore 21 is aligned parallel to the longitudinal axis of the shaft 17 and the optics thus look directly in the distal direction, the working channel 20 is inclined slightly downward in the shaft tip 19, causing the opening of the working channel 20 to appear as oval in Fig. 2. This separation of the bore 21 from the working channel 20 by the dividing wall 22 is confined exclusively to the shaft tip 19, i.e., the interior of the shaft 17 is essentially empty and the optic is initially free to be inserted into the shaft.

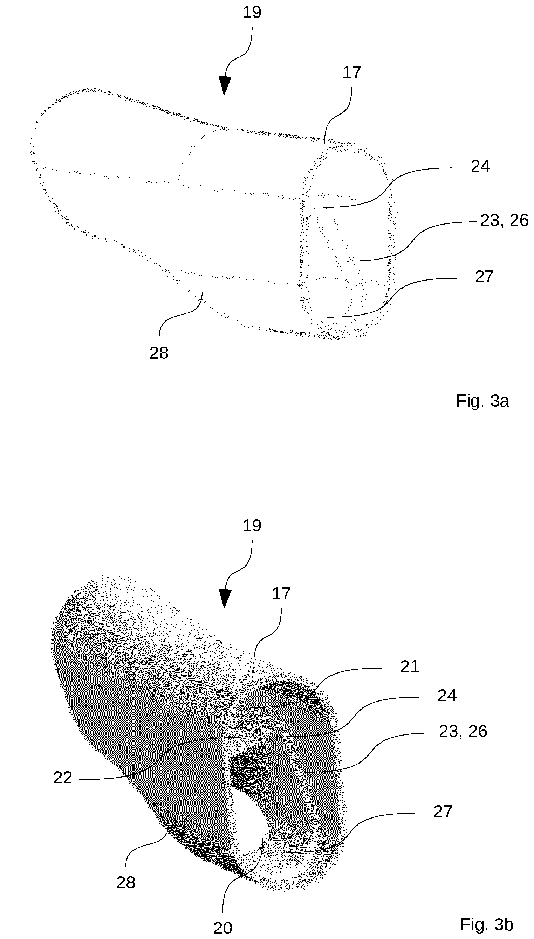

It can be seen from Figs. 3a and 3b that a bar 23 is arranged in front of the bore 21 within the shaft 17. In the embodiment example of the shaft 17 shown here, the bar 23 is U-shaped, with the two free ends 24, 25 of the U oriented in the direction of the bore 21. The bar 23 is oriented at an angle relative to the longitudinal axis of the shaft 17 so that it forms a ramp in the direction of the bore 21 (Fig. 5). The width of the bar 23 or the legs can be a few tenths of a millimeter up to 1 or 2 mm. In addition, it is provided according to the invention that the bar 23 is preferably concave, i.e. that the end face 26 of the bar 23 is curved. The two free ends 24, 25 of the bar end directly in the bore 21 for the optics. Furthermore, the bar 23 also forms the end face 26 of a widening 27 of the wall 28 of the shaft 17 at the distal end 18.

The distance between the free ends 24, 25 of the bar 23 or the distance between the two legs of the U is smaller than the diameter of the optic. As a result, the distal end of the optic is guided in a ramp-like manner over the bar 23 into the bore 21 when the optic is pushed into the shaft 17. Ultimately, this provides a constraining guide for the optic. There is no other way for the optic to be pushed into the shaft 17 than to slide into the bore 21. This positioning of the optics in the bore 21 fixes the optics in this position. This automatically forms the working channel 20 below the optics.

It can be seen from Fig. 4 that the dividing wall 22 is hyperbolic or concave in shape in the direction of the working channel 20. This concave shape of the dividing wall 22 causes the through article passing through the working channel 20 to be directed into the distal open end of the working channel 20. Again, the through article ultimately has no other option but to slide through the working channel 20.

Another essential feature of the invention is that the distance between the free ends 24, 25 of the bar 23 or the distance between the two legs of the U's is greater than the diameter of the through article. This results in the through article, when it encounters the bar 23 during insertion into the shaft 17, being diverted to the center of the working channel 20 and not immediately encountering the opposite leg of the bar 23. Another feature that serves to guide the through article in the proper direction is that the end face 26 of the bar 23 is concavely curved. This curvature offers the particular advantage of preventing the through article from becoming entangled within the working channel 20. Also, when the through article is pulled out of the working channel 20, the widening 27 of the wall 28 and the bar 23 behave in such a way that jamming or snagging can be ruled out.

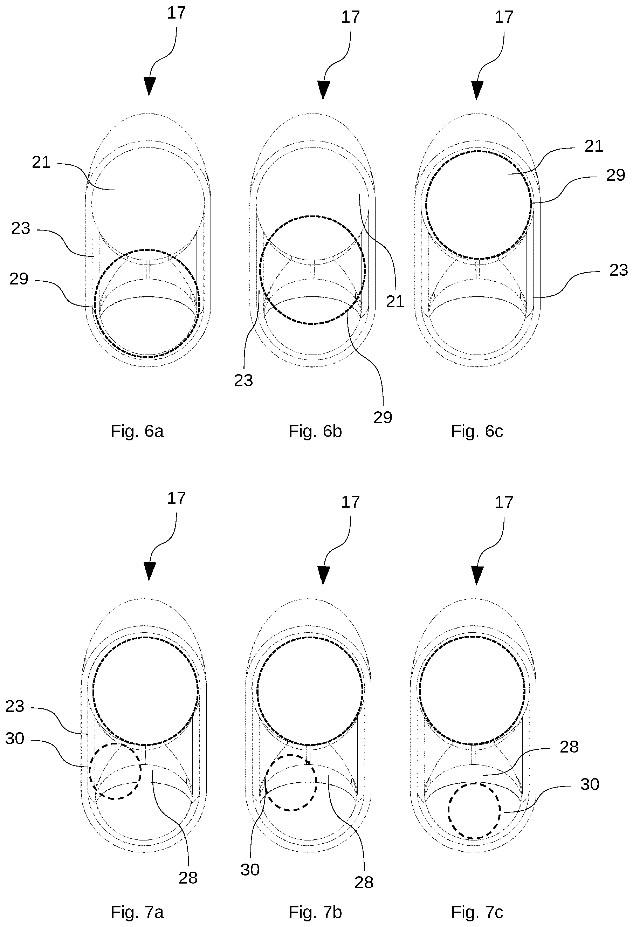

Figs. 6a to 6c and 7a to 7c schematically illustrate how the optic and the through article are guided into the correct positions by the features of the invention. The above figures show a sectional view through the shaft tip 19 according to Fig. 2. Fig. 6a shows the situation where the highly schematized tube-like optic 29 is inserted into the shaft 17 and is not yet fixed in its final position. In this case, the position during insertion into the shaft 17 turns out to be rather random. In the case shown here, the optic 29 is located in the lower area of the shaft 17. When the optic 29 is pushed further in the direction of the distal end 18 of the shaft 17, it meets the bar 23. Due to the ramp-like inclination of the bar 23, the optic 29 is automatically guided into the bore 21 (Fig. 6b, Fig. 6c). In this position shown in Fig. 6c, the optic 29 is fixed. Due to this fixation of the optic 29, the working channel 20 is formed below the optic 29.

As soon as the optic 29 is fixed within the bore 21, the through article 30, which is also shown in a highly schematized manner in Figs. 7a to 7c, can be guided into the working channel 20. Here, too, Fig. 7a first describes a situation which is actually critical for the insertion of the through article 30 into the working channel 20.

The distal end of the through article 30 meets the bar 23, but because the bar is concave, the through article 30 is directed toward the center of the working channel 20 as shown in Fig. 7b. As already explained, another essential feature is that the distance between the legs of the bar 23 is greater than the diameter of the through article 30. When the through article 30 is further inserted into the working channel 20, the through article 30 encounters the partition 22 (Fig. 7b). Since this partition 22 also has a curvature, the through article 30 is guided through the working channel 20 or diverted downward toward the opening of the working channel 20 (Fig. 7c). By means of the features according to the invention, both the optics 29 and the through article 30 are precisely guided into the predetermined positions within the shaft 17 in a simple as well as very reliable manner. Any risk of the optic 29 or the through article 30 becoming jammed when inserted into the shaft or withdrawn from the shaft 17 is prevented by the aforementioned features.

### List of reference signs:

- 10: Hand-held surgical instrument
- 11: Device body
- 12: Proximal end
- 13: Ocular
- 14: Optics
- 15: Connection
- 16: Connection
- 17: Shaft
- 18: Distal end
- 19: Shaft tip
- 20: Working channel
- 21: Bore
- 22: Dividing wall
- 23: Bar
- 24: Free end
- 25: Free end
- 26: Front face
- 27: Widening
- 28: Wall
- 29: Optics
- 30: Through articles

## Claims

1. Shaft (17) for a hand-held surgical instrument (10), in particular for a cystoscope, for receiving an optical system and at least one further through article, preferably a catheter, wherein a proximal end (12) of the tubularly formed shaft (17) can be coupled to a device body (11) and/or a handle unit of the hand-held surgical instrument (10), and a shaft tip (19) at a distal end (18) of the shaft (17) has a bore (21) and a working channel (20) arranged next to the bore (21), wherein the bore (21) is configured to receive the optics and the working channel (20) is configured to receive the through article, whereby at least one bar (23) is arranged on an inner wall of the shaft (17) through which the optics is guided from the proximal end (12) of the shaft (17) into the bore of the shaft tip (19), **characterized in that** the at least one bar (23) is oriented obliquely, in particular ramp-like, with respect to a longitudinal axis of the shaft (17) in the direction of the bore (21).

2. Shaft (17) for a hand-held surgical instrument (10) according to claim 1, **characterized in that** two opposing bars (23) are arranged on the inner wall of the shaft (17).

3. Shaft (17) for a hand-held surgical instrument (10) according to claim 1, **characterized in that** the bar (23) extends in a U-shape along the inner wall of the shaft (17), the open legs of the bar (23) pointing in the direction of the bore (21).

4. Shaft (17) for a hand-held surgical instrument (10) according to one of the preceding claims, **characterized in that** the bar (23) also forms the end face (26) of a widening (27) of the wall (28) of the shaft (17) at the distal end (18) and the end face (26) of the bar (23) is concavely curved.

5. Shaft (17) for a hand-held surgical instrument (10) according to one of the preceding claims, **characterized in that** the distance between the two bars (23) or between the two legs of the U-shaped bar (23) is 1 mm to 4 mm, in particular 2 mm to 4 mm, preferably 3.6 mm, or smaller than a diameter of the optics.

6. Shaft (17) for a hand-held surgical instrument (10) according to one of the preceding claims, **characterized in that** the distance between the two bars (23) or between the two legs of the U-shaped bars (23) is greater than a diameter of the through article.

7. Shaft (17) for a hand-held surgical instrument (10) according to any of the preceding claims, **characterized in that** a dividing wall (22) between the bore (21) and the working channel (20) separates the two openings from each other and the dividing wall (22) is confined exclusively to the shaft tip (19) and the working channel (20) is inclined slightly downward in the shaft tip (19).

8. Shaft (17) for a hand-held surgical instrument (10) according to any one of the preceding claims, **characterized in that** the at least one bar (23) is an end face (26) of a widening (27) of the wall (28) of the shaft tip (19).

9. Shaft (17) for a hand-held surgical instrument (10) according to any one of the preceding claims, **characterized in that** a wall thickness of the working channel (20) is greater than a wall thickness of the bore (21) and/or the shaft (17).

10. Shaft (17) for a hand-held surgical instrument (10) according to any one of the preceding claims, **characterized in that** a cross-section of the shaft (17) is oval or teardrop-shaped.

11. Hand-held surgical instrument (10), in particular cystoscope, resectoscope or the like, having a shaft (17) according to at least one of claims 1 to 10.

## Patentansprüche

1. Schaft (17) für ein chirurgisches Handinstrument (10), insbesondere für ein Zystoskop, zur Aufnahme einer Optik und mindestens eines weiteren Durchlassteils, vorzugsweise eines Katheters, wobei ein proximales Ende (12) des rohrförmig ausgebildeten Schafts (17) mit einem Vorrichtungskörper (11) und/oder einer Griffeinheit des chirurgischen Handinstruments (10) koppelbar ist und eine Schaftspitze (19) an einem distalen Ende (18) des Schafts (17) eine Bohrung (21) und einen neben der Bohrung (21) angeordneten Arbeitskanal (20) aufweist, wobei die Bohrung (21) dazu ausgelegt ist, die Optik aufzunehmen, und der Arbeitskanal (20) dazu ausgelegt ist, das Durchlassteil aufzunehmen, wobei an einer Innenwand des Schafts (17) mindestens ein Steg (23) angeordnet ist, durch den die Optik vom proximalen Ende (12) des Schafts (17) in die Bohrung der Schaftspitze (19) geführt wird, **dadurch gekennzeichnet, dass** der mindestens eine Steg (23) schräg, insbesondere rampenförmig, zu einer Längsachse des Schafts (17) in Richtung der Bohrung (21) ausgerichtet ist.

2. Schaft (17) für ein chirurgisches Handinstrument (10) nach Anspruch 1, **dadurch gekennzeichnet, dass** an der Innenwand des Schafts (17) zwei gegenüberliegende Stege (23) angeordnet sind.

3. Schaft (17) für ein chirurgisches Handinstrument (10) nach Anspruch 1, **dadurch gekennzeichnet, dass** sich der Steg (23) U-förmig entlang der Innenwand des Schafts (17) erstreckt, wobei die offenen Schenkel des Stegs (23) in Richtung der Bohrung (21) weisen.

4. Schaft (17) für ein chirurgisches Handinstrument (10) nach einem der vorhergehenden Ansprüche, **dadurch gekennzeichnet, dass** der Steg (23) außerdem die Stirnfläche (26) einer Aufweitung (27) der Wand (28) des Schafts (17) am distalen Ende (18) ausbildet und die Stirnfläche (26) des Stegs (23) konkav gekrümmt ist.

5. Schaft (17) für ein chirurgisches Handinstrument (10) nach einem der vorhergehenden Ansprüche, **dadurch gekennzeichnet, dass** der Abstand zwischen den beiden Stegen (23) oder zwischen den beiden Schenkeln des U-förmigen Stegs (23) 1 mm bis 4 mm, insbesondere 2 mm bis 4 mm, vorzugsweise 3,6 mm, beträgt oder kleiner als der Durchmesser der Optik ist.

6. Schaft (17) für ein chirurgisches Handinstrument (10) nach einem der vorhergehenden Ansprüche, **dadurch gekennzeichnet, dass** der Abstand zwischen den beiden Stegen (23) oder zwischen den beiden Schenkeln der U-förmigen Stege (23) größer als der Durchmesser des Durchlassteils ist.

7. Schaft (17) für ein chirurgisches Handinstrument (10) nach einem der vorhergehenden Ansprüche, **dadurch gekennzeichnet, dass** eine Trennwand (22) zwischen der Bohrung (21) und dem Arbeitskanal (20) die beiden Öffnungen voneinander trennt und die Trennwand (22) ausschließlich auf die Schaftspitze (19) beschränkt ist und der Arbeitskanal (20) in der Schaftspitze (19) leicht nach unten geneigt ist.

8. Schaft (17) für ein chirurgisches Handinstrument (10) nach einem der vorhergehenden Ansprüche, **dadurch gekennzeichnet, dass** der mindestens eine Steg (23) eine Stirnfläche (26) einer Aufweitung (27) der Wand (28) der Schaftspitze (19) ist.

9. Schaft (17) für ein chirurgisches Handinstrument (10) nach einem der vorhergehenden Ansprüche, **dadurch gekennzeichnet, dass** die Wandstärke des Arbeitskanals (20) größer als die Wandstärke der Bohrung (21) und/oder des Schafts (17) ist.

10. Schaft (17) für ein chirurgisches Handinstrument (10) nach einem der vorhergehenden Ansprüche, **dadurch gekennzeichnet, dass** der Querschnitt des Schafts (17) oval oder tropfenförmig ist.

11. Chirurgisches Handinstrument (10), insbesondere Zystoskop, Resektoskop oder dergleichen, mit einem Schaft (17) nach mindestens einem der Ansprüche 1 bis 10.

## Revendications

1. Tige (17) pour un instrument chirurgical à main (10), en particulier pour un cystoscope, destinée à recevoir un système optique et au moins un autre article traversant, de préférence un cathéter, une extrémité proximale (12) de la tige tubulaire (17) pouvant être couplée à un corps de dispositif (11) et/ou à une unité de poignée de l'instrument chirurgical à main (10), et une pointe de tige (19) au niveau d'une extrémité distale (18) de la tige (17) comportant un alésage (21) et un canal de travail (20) agencé à côté de l'alésage (21), l'alésage (21) étant configuré pour recevoir l'optique et le canal de travail (20) étant configuré pour recevoir l'article traversant, au moins une barre (23) étant agencée sur une paroi intérieure de la tige (17) à travers laquelle l'optique est guidée depuis l'extrémité proximale (12) de la tige (17) dans l'alésage de la pointe de tige (19), **caractérisée en ce que** l'au moins une barre (23) est orientée obliquement, en particulier en forme de rampe, par rapport à un axe longitudinal de la tige (17) en direction de l'alésage (21).

2. Tige (17) pour un instrument chirurgical à main (10) selon la revendication 1, **caractérisée en ce que** deux barres opposées (23) sont agencées sur la paroi intérieure de la tige (17).

3. Tige (17) pour un instrument chirurgical à main (10) selon la revendication 1, **caractérisée en ce que** la barre (23) s'étend en forme de U le long de la paroi intérieure de la tige (17), les branches ouvertes de la barre (23) étant dirigées vers l'alésage (21).

4. Tige (17) pour un instrument chirurgical à main (10) selon l'une des revendications précédentes, **caractérisée en ce que** la barre (23) forme également la face frontale (26) d'un élargissement (27) de la paroi (28) de la tige (17) à l'extrémité distale (18) et la face frontale (26) de la barre (23) est incurvée de manière concave.

5. Tige (17) pour un instrument chirurgical à main (10) selon l'une des revendications précédentes, **caractérisée en ce que** la distance entre les deux barres (23) ou entre les deux branches de la barre en U (23) est de 1 mm à 4 mm, en particulier de 2 mm à 4 mm, de préférence de 3,6 mm, ou inférieure à un diamètre de l'optique.

6. Tige (17) pour un instrument chirurgical à main (10) selon l'une des revendications précédentes, **caractérisée en ce que** la distance entre les deux barres (23) ou entre les deux branches des barres en U (23) est supérieure à un diamètre de l'article traversant.

7. Tige (17) pour un instrument chirurgical à main (10) selon l'une quelconque des revendications précédentes, **caractérisée en ce qu'**une paroi de séparation (22) entre l'alésage (21) et le canal de travail (20) sépare les deux ouvertures l'une de l'autre et la paroi de séparation (22) est limitée exclusivement à la pointe de tige (19) et le canal de travail (20) est légèrement incliné vers le bas dans la pointe de tige (19).

8. Tige (17) pour un instrument chirurgical à main (10) selon l'une quelconque des revendications précédentes, **caractérisée en ce que** l'au moins une barre (23) est une face frontale (26) d'un élargissement (27) de la paroi (28) de la pointe de tige (19).

9. Tige (17) pour un instrument chirurgical à main (10) selon l'une quelconque des revendications précédentes, **caractérisée en ce qu'**une épaisseur de paroi du canal de travail (20) est supérieure à une épaisseur de paroi de l'alésage (21) et/ou de la tige (17).

10. Tige (17) pour un instrument chirurgical à main (10) selon l'une quelconque des revendications précédentes, **caractérisée en ce qu'**une section transversale de la tige (17) est ovale ou en forme de goutte d'eau.

11. Instrument chirurgical à main (10), en particulier cystoscope, réectoscope ou analogue, comportant une tige (17) selon au moins l'une des revendications 1 à 10.
